# EUROPÄISCHE PATENTSCHRIFT

(11) **EP 2 190 425 B1**
(45) Veröffentlichungstag und Bekanntmachung des Hinweises auf die Patenterteilung: **07.10.2015**
(21) Anmeldenummer: 08734983.3
(22) Anmeldetag: 03.04.2008
(51) Int. Cl.: A61K 31/22, A61K 31/225, A61Q 3/00, A61P 31/10, A61K 47/10, A61K 9/00

(54) **TOPISCH ANWENDBARE FUNGIZIDE MITTEL ZUR NAGELBEHANDLUNG**
TOPICALLY APPLICABLE FUNGICIDE AGENTS FOR TREATING NAILS
AGENTS FONGICIDES À USAGE TOPIQUE POUR LE TRAITEMENT DES ONGLES

(30) Priorität: 20.04.2007 CH 656072007
(43) Veröffentlichungstag der Anmeldung: 02.06.2010
(73) Patentinhaber: BioEqual AG, 4132 Muttenz (CH)
(72) Erfinder: MEYER, Hans, CH-4125 Riehen (CH)
(74) Vertreter: Foraita, Hans-Günther
(86) Internationale Anmeldenummer: PCT/EP2008/002642
(87) Internationale Veröffentlichungsnummer: WO 2008/128627

(56) Entgegenhaltungen:
- EP-A1- 0 503 988
- EP-A2- 1 143 950
- WO-A-00/15202
- WO-A-99/39680
- WO-A-2004/032886
- WO-A1-96/11572
- WO-A2-2004/021964
- GB-A- 1 234 297
- GB-A- 1 561 475
- US-A- 3 806 513
- US-A- 3 806 593
- US-A1- 2004 062 733
- US-A1- 2005 020 678
- US-A1- 2010 113 593
- EPODOC HOST- EPODOC, 10. Juni 1998 (1998-06-10), XP002124817 & CN 1 183 961 A (NO 1 HOSPITAL ATTACHED TO SUZH [CN]) 10. Juni 1998 (1998-06-10)
- FAERGEMANN J ET AL: "Treatment of onychomycosis with a propylene glycol-urea-lactic acid solution.", MYCOSES OCT 1989 LNKD- PUBMED:2531288, vol. 32, no. 10, October 1989 (1989-10), pages 536-540, ISSN: 0933-7407

## Beschreibung

Die vorliegende Erfindung betrifft.

Ein oder gegebenenfalls mehrere C1-C4 Alkylester der Milchsäure, Aepfelsäure, Weinsäure oder Zitronensäure zur Verwendung in der Behandlung von Nagelerkrankungen verursacht durch Dermatophyten, wobei der oder gegebenenfalls mehrere C1-C4 Alkylester in einem topisch anwendbaren wasserfreien Mittel, welches gegebenenfalls physiologisch verträgliche Hilfsstoffe enthält, vorliegt.

In WO 00/15202 werden topisch verwendbare Mittel beschrieben, die eine oder mehrere Wirksubstanzen neben Trägerstoffen (Carrier), darunter Säureester und gegebenenfalls physiologisch verträgliche Hilfsstoffe enthalten.

Ueberraschenderweise wurde gefunden, dass die nur einen C1-C4-Alkylester der Milchsäure, der Aepfelsäure, der Weinsäure oder der Zitronensäure oder Gemische davon ohne weitere Zusatzstoffe enthaltenen Mittel zur Behandlung von Nagelerkrankungen, die auf Mykosenbefall (Pilzbefall) beruhen, hervorragend geeignet sind.

Ueberraschenderweise können diese topisch antimykotischen Mittel ohne Zugabe von weiteren Wirkstoffen und ohne Hinzufügung von weiteren Trägerstoffen (Carrier) äusserst wirksam verwendet werden.

Aus dem bisher bekannten Stand der Technik ist die bakterizide Wirkung der C1-C4-Alkylester der Milchsäure, der Aepfelsäure der Weinsäure oder Citronensäure hinlänglich bekannt.

Beispielsweise wird in der GBP 1234297 die fungizide Wirkung der Niederalkylester der Milchsäure, der Aepfelsäure, der Weinsäure oder der Citronensäure weder beschrieben und auch nicht in Begleitung anderer Wirkstoffe erwähnt. Die verschiedenen Milchsäureester werden auch als bakterizide Mittel nicht erwähnt.

In der USP-3806513 werden Milchsäureester mit niederen Alkanolen als Trägerstoffe (Carrier), wirksam zur Behandlung von Acne, Pityriasis und Oleosa Capitis beschrieben. Die fungizide bzw. antimykotische wird

nicht erwähnt, nur die bakterizide Wirkung. Die Ester werden in Begleitung von Alkoholen in alkoholischer Lösung als Träger verwendet.

In der GBP 156 1475 wird die Herstellung von bakteriostatischen Lösungen, verwendbar als Deodorant beschrieben, wobei die freie Milchsäure in alkoholischer Lösung vorliegt. Milchsäureester werden nicht erwähnt, jedoch die Hydrolisierung dieser Ester. Es ist darauf zu schliessen, dass ein Gleichgewicht vorliegt. Eine fungizide Wirkung der Lösungen wird nicht beschrieben. Es wird die Vermutung geäussert, dass ein Teil der bakteriziden Wirkung dem verwendeten Alkohol zuzuschreiben ist.

Die USP-2005019355 beschreibt Zusammensetzungen, die Bakterien auf der Hand reduzieren, welche Ester von Milchsäure enthalten und insbesondere bakteriostatisch wirksam sind. Die Ester der Milchsäure sind in verschiedenen Lösungsmitteln gelöst. Eine fungizide Wirkung wird keineswegs beschrieben oder sogar beansprucht.

Die WO 2004032886 beschreibt die Verwendung von Salzen der Milch- bzw. Citronensäure als bakterizide Mittel. Insbesondere wird die Verwendung dieser Mittel auf der Haut und insbesondere für die Haare zur Bekämpfung von Bakterien beschrieben. Fungizide Wirkungen werden nicht erwähnt und beansprucht. Die Verwendung von Surfactants (oberflächenaktiven Mitteln) und insbesondere die Verwendung einer zweiten Säure wird als vorteilhaft beschrieben.

US2005/0020678 beschreibt eine fungizide Zusammensetzung die Alkylester der Milchsäure enthält. Die Zusammensetzung wird insbesondere zum topischen Gebrauch bei Pflanzen, Tieren und zur Oberflächendesinfektion verwendet. In Absatz 8 wird die direkte topische Anwendung von 2-5% Ethyllaktat beim Menschen beschrieben. Die Anwendung bei Nagelerkrankungen wird nicht beschrieben. Die getesteten Pilze, Penicillum und Aspergillus sind keine Dermatophythen.

WO96/11572 beschreibt antimikrobielle Zusammensetzungen die Glykole und Carbonsäuren enthalten. Auf Seite 11 wird die fungizide Wirkung gegen Trichophython rubrum von Milchsäure, Essigsäure, Weinsäure und Zitronensäure belegt (MIC-Werte). Die Ester dieser Säuren werden nicht erwähnt.

In keiner der erwähnten Patentschriften wird auf die fungizide Wirkung der Ester der Milch-, Aepfel-, Wein- oder Citronensäure verwiesen, die zur Behandlung von Nagelbetterkrankungen durch Pilzbefall geignet sind.

Ueberraschenderweise können die C1-C4-Alkylester als fungizide Wirkstoffe und gleichzeitig auch als Carrier in eigenem Bedarfsfall verwendet werden, die keinen weiteren Zusatzstoff erforderlich machen.

Es gibt bisher kein zufriedenstellendes Mittel zur topischen Behandlung von Nagelerkrankungen, verursacht durch Mykosen (Pilze), welch entweder ohne zusätzlichen Carrier bzw. ohne zusätzlichen Wirkstoff Verwendung findet. Die C1-C4-Alkylester der Milchsäure, der Aepfelsäure, Weinsäure oder auch Citronensäure weisen überraschenderweise gute antimykotische und gleichzeitig gute Eigenschaften als Carrier zum eigenen Zweck auf, welche als kosmetische Mittel zur topischen Behandlung von Nagelerkrankungen, d.h. den Transport eines C1-C4-Alkylesters der entsprechenden Säuren für einen dauerhaften Behandlungserfolg erforderliche Menge an Wirksubstanz durch den Nagel in das darunter liegende Nagelbett und zur Nagelwurzel (Matrix) gewährleistet.

Die als Wirkstoff und gleichzeitig als Carrier zu verwendenden C1-C4-Alkylester umfassen die Methyl-, Aethyl-, n-Propyl-, Isopropyl-, n-Butyl-, sec. Butyl-, Isobutyl- und tert.-Butylester. Bei den Estern der mehrbasischen Säuren Aepfelsäure, Citronensäure und Weinsäure können die in den Estergruppen enthaltenen C1-C4-Alkylgruppen gleich oder verschieden sein. In den vorgenannten mehrbasischen Säuren können alle Carboxygruppen oder ein Teil der Carboxygruppen verestert sein. Es können daher neben Aepfel- und Weinsäure-C1-C4-alkylester auch die entsprechenden Aepfel- und Weinsäure-C 1-C4-dialkylestren auch die entsprechenden Aepfel- und Weinsäure monoalkylester in Betracht. Von den C1-C4-Alkylestern der Citronensäure sind die entsprechenden Mono-, Di- und Trialkylestern geeignet.

Bevorzugte Ester sind die Ethylester. Weitere bevorzugte Ester die Isopropylester.

Eine bevorzugte Einzelverbindung ist der Milchsäureethylester. Weitere bevorzugte Einzelverbindungen sind der Aepfelsäurediethylester und Aepfelsäuredisopropylester . Desgleichen sind der Weinsäuremonoethylester und der Citronensäuremonoethylester als bevorzugte Verbindungen zu bezeichnen.

Die erfindungsgemässen topisch anwendbaren Mittel können neben einem oder mehreren C1-C4- Alkylestern der Milchsäure, der Aepfelsäure, der Weinsäure oder der Citronensäure übliche physiologisch verträgliche Hilfsstoffe enthalten.

Geeignete Hilfsstoffe dieser Art sind beispielsweise Terpene oder Terpene enthaltende Öle, Alkohole, Ketone, Fettsäureester, Polyglykole, Tenside, Harnstoff, Antioxidantien und Komplexierungsmittel.

Geeignete Terpene sind acyclische, monocyclische und bicyclische Terpene sowie Öle, die diese Terpene enthalten. Beispiele für acyclische Terpene sind acyclische Terpenkohleilwasserstoffe, wie z.B. Myrcen, acyclische Terpenalkohole, wie z.B. Citronellol und Geraniol, sowie acyclische Terpenaldehyde und -ketone, wie z.B. Citral, α-Jonon und β-Jonon. Beispiele für monocyclische Terpene sind monocyclische Terpenkohlenwasserstoffe, wie z.B. α-Terpinen, γ-Terpinen und Limonen, monocyclische Terpenalkohole, wie z.B. Thymol, Menthol, Cineol und Carvacrol, sowie monocyclische Terpenketone, wie z.B. Menthon und Carvon. Beispiele für bicyclische Terpene sind Terpene aus der Carangruppe, wie z.B. Caron, Terpene aus der Pinangruppe, wie z.B. α-Pinen und β-Pinen, sowie Terpene aus der Bornangruppe, wie z.B.Campher und Borneol. Besonders geeignete Terpene sind monocyclische Terpenalkohole, wie z.B. Thymol und Menthol. Beispiele für geeignete Öle, die Terpene enthalten, sind Pfefferminzöl, Cardamomenöl, Geraniumöl, Rosenöl, Thujaöl und Thymianöl. Besonders geeignete Öle sind Pfefferminzöl, Lavendelöl und Thymianöl.

Geeignete Alkohole sind verzweigte oder unverzweigte Alkohole mit 1 bis 3 Hydroxygruppen und 2 bis 6 Kohlenstoffatomen, wobei die Hydroxygruppen teilweise oder vollständig verethert und verestert sein können. Speziell geeignete Alkohole sind Ethanol, 1-Propanol, 2-Propanol (Isopropanol), 1,2-Propandiol (Propylenglykol), 2-Phenylethanol (Phenylethylalkohol), 1-Butanol (Butylalkohol), Ethylenglycolmonomethylether (Methoxyethanol), Ethylenglycolmonophenylether (Phenoxyethanol), 1,2,3-Trihydroxypropan (Glycerin), Ethylacetat, Butylacetat, Glycerindiacetat (Diacetin) und Glycerintriacetat (Triacetin).

Als geeignete Ketone kommen beispielsweise Aceton und Methylethylketon
(2-Butanon) in Betracht.

Als Fettsäureester sind Ester von gesättigten und ungesättigten, verzweigten und unverzweigten Fettsäuren mit 8 bis 21 Kohlenstoffatomen geeignet, wobei die Alkoholkomponente verzweigte und unverzweigte Alkohole mit 1 bis 6 Kohlenstoffatomen umfasst.

Speziell geeignete Fettsäureester sind Tridecancarbonsäureisopropylester, Tetradecancarbonsäureisopropylester (Isopropylmyristat), Pentadecancarbonsäuremethylester und 9-Octadecensäureglycerinmonoester (Glycerinmonooleat).

Ein geeignetes Polyglykol ist beispielsweise Polyglykol 400.

Geeignete Tenside sind beispielsweise nicht ionogene oberflächenaktive Substanzen. Speziell geeignete Tenside sind Partialfettsäureester des Sorbitans (Span), Partialfettsäureester des Polyoxyethylensorbitans (Tween), Fettsäureester des Polyoxyethylens (Myrj) und Fettalkoholether des Polyoxyethylens (Brij).

Geeignete Antioxidantien sind beispielsweise Butylhydroxytoluol (BHT), Butyl-4-methoxyphenol (BHA), Tocopherole und Ascorbate.

Als Komplexierungsmittel eignen sich beispielsweise Ethylendiamintetraessigsäure (EDTA) und Dinatrium-Ethylendiamintetraessigsäure (Na₂-ETDA).

Als erfindungsgemässe topisch anwendbare Mittel konnten zum Beispiel wasserfreie Lösungen, Tinkturen, Emulsionen, Gele, Salben, Cremes und Pasten in Betracht. Bevorzugte topische Darreichungsformen sind wasserfreie Lösungen. Die erhaltene Lösung wird bevorzugt direkt als solche zur topischen Applikation verwendet.

Die erhaltene wasserfreie Lösung kann aber auch unter Zugabe von weiteren physiologisch verträglichen Formulierungshilfstoffen mittels konventionellen Lösungs, Misch und Suspendierungsverfahren in eine Andere topische Darreichungsform gebracht werden. Die erfindungsgemässen topisch anwendbaren Mittel sind in Form von wasserfreien Lösungen.

Bevorzugte topisch anwendbare Mittel enthalten gemäss vorliegender Erfindung

1- 99,9 Gew.-% eines oder mehrerer C1-C4-Alkylester der Milchsäure, der Aepfelsäure, der Weinsäure oder der Citronensäure und 0 bis 98,99 Gew.-% eine oder mehrerer physiologisch verträglicher Hilfsstoffe.

Die Erfindung betrifft ein oder gegebenenfalls mehrere C1-C4 Alkylester der Milchsäure, Aepfelsäure, Weinsäure oder Zitronensäure zur Verwendung in der Behandlung von Nagelerkrankungen verursacht durch Dermatophyten, wobei der oder gegebenenfalls mehrere C1-C4 Alkylester in einem topisch anwendbaren wasserfreien Mittel, welches gegebenenfalls physiologisch verträgliche Hilfsstoffe enthält, vorliegt. Insbesondere betrifft die Erfindung die Behandlung von Pilzinfektionen, durch Dermatophyten wie zum Bespiel Trichophyton mentagroph befallende Finger-oder Fussnägel. Ferner können die erfindungsgemässen Mittel auch zur Behandlung von Pilzinfektionen durch Dermatophyten von Hufen, Klauen und Krallen von Haus- und Nutztieren und in Gefangenschaft lebender Wildtiere verwendet werden.

Typisch anwendbare Mittel, die einen C1-C4-Alkylester der genannten Säuren enthalten eignen sich als Antimykotika beispielsweise zur
- Behandlung, und Nachbehandlung von Onychomykosis verursacht durch Dermatophyten
- Behandlung, und Nachbehandlung von Nagelpilzinfektionen durch Dermatophyten bei Patienten mit Psoriasis, Diabetes oder auch AIDS,
- Unterstützung der Behandlung von periungualen Nagelinfektionen wie Trychophyton mentagroph.

Wie bereits gesagt eignen sich die erfindungsgemässen pharmazeutischen Mittel zur Behandlung von Nagelerkrankungen und periungualen Erkrankungen an Zehen- und Fingernägeln, sowie zur Behandlung der Hufe, Klauen und Krallen von Haus- und Nutztieren und Wildtieren in Gefangenschaft (Zoo), die durch Dermatophyten verusacht wurden.

Die Applikationsfrequenz der erfindungsgemässen Mittel richtet sich nach dem Ausmass und der Lokalisation der Erkrankungen.

Im allgemeinen genügt eine ein bis dreimalige Anwendung pro Tag.

Die wasserfreie Lösung wird hierbei direkt auf den kranken Nagel, bzw. dem Huf, die Klaue oder die Kralle und bei Bedarf auch auf die ebenfalls befallenden umliegenden Hautsegmente mittels Pipette oder Applikator aufgetragen.

Die erfindungdgemässen topisch anwendbaren Mittel haben den Vorteil, dass sie den erkrankten Nagel innerhalb von wenigen Tagen durchdringen und im Nagelbett oder Nagelwurzel ihre Wirkung voll entfalten können.

### Beispiel 1

Nagelapplikator enthaltend
Milchsäureethylester, rein ohne Zusatz.

### Beispiel 2

Nagelapplikator enthaltend

| | |
|---|---|
| Aepfelsäurediethylester | 70% |
| Ethanol | 30%, |

Keine weiteren Zusätze.

### Beispiel 3

Nagelapplikator enthaltend

| | |
|---|---|
| Citronensäuretriethylester | 50% |
| Isopropanol | 50%. |

Keine weiteren Zusätze.

### Beispiel 4

Nagelapplikator enthaltend

| | |
|---|---|
| Weinsäurediethylester | 40% |
| Ethanol | 30% |
| Propylenglycol | 30% |

Keine weiteren Zusätze.

Als mögliche Applikatoren können beispielsweise folgende eingesetzt werden:
1) Applikatoren, welche auf ein Kapillarsystem beruhen, vergleichbar mit Textl-Linern, welche aus entsprechend resistenten Materialien wie z.B. Polypropylen gefertigt sind, beschrieben in DE 3202435C1 bzw. USP 4.973.181.
2) Tupferflaschen mit selbstätigem Feder- oder Rollverschluss wie z.B Dab-O-Matic der Firma Dab-O-Matic Corp. in Mount Vernon, N Y (USA), oder
3) Uebliche Tinkturflaschen aus Glas oder Kunststoff mit im Verschluss eingebauten Pinsel oder Pipette.

## Patentansprüche

1. Ein oder gegebenenfalls mehrere C1-C4 Alkylester der Milchsäure, Aepfelsäure, Weinsäure oder Zitronensäure zur Verwendung in der Behandlung von Nagelerkrankungen verursacht durch Dermatophyten, wobei der oder gegebenenfalls mehrere C1-C4 Alkylester in einem topisch anwendbaren wasserfreien Mittel, welches gegebenenfalls physiologisch verträgliche Hilfsstoffe enthält, vorliegt.

2. Ein oder gegebenenfalls mehrere C1-C4 Alkylester der Milchsäure, Aepfelsäure, Weinsäure oder Zitronensäure zur Verwendung nach Anspruch 1, **dadurch gekennzeichnet, dass** man einen Ethylester der Milchsäure, Aepfelsäure, der Weinsäure oder der Zitronensäure verwendet.

3. Ein oder gegebenenfalls mehrere C1-C4 Alkylester der Milchsäure, Aepfelsäure, Weinsäure oder Zitronensäure zur Verwendung nach Anspruch 1, **dadurch gekennzeichnet, dass** man einen Isopropylester der Milchsäure, Aepfelsäure, Weinsäure oder der Zitronensäure verwendet.

4. Ein oder gegebenenfalls mehrere C1-C4 Alkylester der Milchsäure, Aepfelsäure, Weinsäure oder Zitronensäure zur Verwendung nach Anspruch 1, **dadurch gekennzeichnet, dass** man Aepfelsäurediisopropylester verwendet.

5. Ein oder gegebenenfalls mehrere C1-C4 Alkylester der Milchsäure, Aepfelsäure, Weinsäure oder Zitronensäure zur Verwendung nach Ansprüche 1-4, **dadurch gekennzeichnet, dass** das Mittel einen oder mehrere Hilfsstoffe aus der Gruppe der Terpene oder Terpene enthaltende Oele, Alkohole, Ketone Fettsäureester, Polyglykolen, Tenside, Harnstoff, Antioxydantien und Komplexierungsmittel enthält.

6. Ein oder gegebenenfalls mehrere C1-C4 Alkylester der Milchsäure, Aepfelsäure, Weinsäure oder Zitronensäure zur Verwendung nach Ansprüche 1-4, **dadurch gekennzeichnet, dass** das Mittel 1 bis 99,99 Gewichtsprozent an C1-C4 Alkylester der Milchsäure, Aepfelsäure, Weinsäure oder der Zitronensäure und 0 bis 98,99% Hilfsstoffe enthält.

## Claims

1. One or, where applicable, more than one C1-C4 alkyl esters of lactic acid, malic acid, tartaric acid or citric acid for use in the treatment of-nail diseases caused by dermatophytes, wherein the C1-C4 alkyl ester or C1-C4 alkyl esters is present in an anhydrous medium for topical application, which comprises where applicable physiologically compatible excipients.

2. One or, where applicable, more than one C1-C4 alkyl esters of lactic acid, malic acid, tartaric acid or citric acid for use according to Claim 1, **characterized by** the fact that an ethyl ester of lactic acid, malic acid, tartaric acid or citric acid is used.

3. One or, where applicable, more than one C1-C4 alkyl esters of lactic acid, malic acid, tartaric acid or citric acid for use according to Claim 1, **characterized by** the fact that an isopropyl ester of lactic acid, malic acid, tartaric acid or citric acid is used.

4. One or, where applicable, more than one C1-C4 alkyl esters of lactic acid, malic acid, tartaric acid or citric acid for use according to Claim 1, **characterized by** the fact that malic acid diisopropyl ester is used.

5. One or, where applicable, more than one C1-C4 alkyl esters of lactic acid, malic acid, tartaric acid or citric acid for use according to Claims 1-4, **characterized by** the fact that the medium contains one or more excipients from the group terpenes or terpene-containing oils, alcohols, ketones, fatty acid esters, polyglycols, surfactants, urea, antioxidants and complexing agents.

6. One or, where applicable, more than one C1-C4 alkyl esters of lactic acid, malic acid, tartaric acid or citric acid for use according to Claims 1-4, **characterized by** the fact that the medium contains 1 to 99.99 percent by weight of C1-C4 alkyl esters lactic acid, malic acid, tartaric acid or citric acid and 0 to 98.99% excipients.

## Revendications

1. Un ou éventuellement plusieurs ester(s) d'alkyle en C1-C4 de l'acide lactique, malique, tartrique ou citrique pour une utilisation dans le traitement des affections unguéales causées par des dermatophytes, ce ou ces ester(s) d'alkyle en C1-C4 étant présent(s) dans un agent anhydre à application topique, contenant éventuellement des excipients physiologiquement tolérables.

2. Un ou éventuellement plusieurs ester(s) d'alkyle en C1-C4 de l'acide lactique, malique, tartrique ou citrique pour une utilisation selon la revendication 1 et caractérisé(s) par l'utilisation d'un ester éthylique de l'acide lactique, malique, tartrique ou citrique.

3. Un ou éventuellement plusieurs ester(s) d'alkyle en C1-C4 de l'acide lactique, malique, tartrique ou citrique pour une utilisation selon la revendication 1 et caractérisé(s) par l'utilisation d'un ester isopropylique de l'acide lactique, malique, tartrique ou citrique.

4. Un ou éventuellement plusieurs ester(s) d'alkyle en C1-C4 de l'acide lactique, malique, tartrique ou citrique pour une utilisation selon la revendication 1 et caractérisé(s) par l'utilisation d'un diisopropyl-ester de l'acide malique.

5. Un ou éventuellement plusieurs ester(s) d'alkyle en C1-C4 de l'acide lactique, malique, tartrique ou citrique pour une utilisation selon les revendications 1-4 et caractérisé(s) par le fait que le produit contient un ou plusieurs excipients du groupe des terpènes ou des huiles contenant des terpènes, alcools, cétones, esters d'acide gras, polyglycols, surfactants, urée, antioxydants et agents complexants.

6. Un ou éventuellement plusieurs ester(s) d'alkyle en C1-C4 de l'acide lactique, malique, tartrique ou citrique pour une utilisation selon les revendications 1-4 et caractérisé(s) par le fait que le produit contient 1 à 99,9 pour cent en poids d'ester(s) d'alkyle en C1-C4 de l'acide lactique, malique, tartrique ou citrique et 0 à 98,99% d'excipients.
